# EUROPEAN PATENT APPLICATION

(11) **EP 0 857 782 A2**
(43) Date of publication of application: **12.08.1998**
(21) Application number: 97309346.1
(22) Date of filing: 20.11.1997
(51) Int. Cl.: C12N 15/12, C12N 5/10, C07K 14/715, C07K 16/28

(54) **Novel mammalian DD-1 gene**

(30) Priority: 27.01.1997 US 789355
(71) Applicant: SMITHKLINE BEECHAM CORPORATION, Philadelphia Pennsylvania 19103 (US)
(72) Inventor: Emery, John G., King of Prussia, Pennsylvania 19406 (US)
(74) Representative: Crump, Julian Richard John

(57) **Abstract**

DD-1 polypeptides and polynucleotides and methods for producing such polypeptides by recombinant techniques are disclosed. Also disclosed are methods for utilizing DD-1 polypeptides and polynucleotides in the design of protocols for the treatment of neurodegenerative disorders (including Alzheimer's disease, Parkinson's disease, cerebellar degeneration, and amyotrophic lateral sclerosis), ischemic injury (including stroke, myocardial infarction, and reperfusion injury), AIDS, aplastic anemia, polycistic kidney disease, osteoporosis, chronic degenerative liver disease, cancer, autoimmune disorders (including systemic lupus erythematosus and immune-mediated glomerulonephritis), viral infections, and rheumatoid arthritis, among others, and diagnostic assays for such conditions.

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to death domain cytoplasmic adaptor family, hereinafter referred to as DD-1. The invention also relates to inhibiting or activating the action of such polynucleotides and polypeptides.

### BACKGROUND OF THE INVENTION

Many biological actions, for instance, response to certain stimuli and natural biological processes, are controlled by factors, such as cytokines. Many cytokines act through receptors by engaging the receptor and producing an intracellular response. For example, tumor necrosis factors (TNF) alpha and beta are cytokines which act through TNF receptors to regulate numerous biological processes, including protection against infection, induction of shock and inflammatory disease, and induction of programmed cell death (or apoptosis). The TNF molecules belong to the "TNF-ligand" superfamily and act together with their receptors or counter-ligands, the "TNF-receptor" superfamily.

Among the ligands there are included TNF-α, lymphotoxin-α (LT-α, also known as THF-β), LT-β (found in complex heterotrimer LT-α2-β), FasL, CD40L, CD27L, CD30L, 4-1BBL, OX40L and nerve growth factor (NGF)). The superfamily of TNF receptors includes the p55TNF receptor, p75TNF receptor, TNF receptor-related protein, FAS antigen or APO-1, CD40, CD27, CD30, 4-1BB, OX40, low affinity p75 and NGF-receptor (Meager, A., Biologicals, 22:291-295 (1994)).

Many members of the TNF-ligand superfamily are expressed by activated T-cells, implying that they are necessary for T-cell interactions with other cell types which underlie cell ontogeny and functions (Meager, A., supra).

Considerable insight into the essential functions of several members of the TNF receptor family has been gained from the identification and creation of mutants that abolish the expression of these proteins. For example, naturally occurring mutations in the Fas antigen and its ligand cause lymphoproliferative disease (Watanabe-Fukunaga, R., et al., Nature 356:314 (1992)), perhaps reflecting a failure of programmed cell death. Mutations of the CD40 ligand cause an X-linked immunodeficiency state characterized by high levels of immunoglubulin M and low levels of immunoglobulin G in plasma, indicating faulty T-cell-dependent B-cell activation (Allen, R.C. et al., Science 259:990 (1993)). Targeted mutations of the low affinity nerve growth factor receptor cause a disorder characterized by faulty sensory innovation of peripheral structures (Lee, K.F. et al, Cell 69:737 (1992)).

TNF and LT-α are capable of binding to two TNF receptors (the 55- and 75-kd TNF receptors). A large number of biological effects elicited by TNF and LT-α, acting through their receptors, include hemorrhagic necrosis of transplanted tumors, cytotoxicity, a role in endotoxic shock, inflammation, immunoregulation, proliferation and anti-viral responses, as well as protection against the deleterious effects of ionizing radiation. TNF and LT-α are involved in the pathogenesis of a wide range of diseases, including endotoxic shock, cerebral malaria, tumors, autoimmuine disease, AIDS and graft-host rejection (Beutler, B. and Von Huffel, C., Science 264:667-668 (1994)). Mutations in the p55 Receptor cause increased susceptibility to microbial infection.

Apoptosis, or programmed cell death, is a physiologic process essential to the normal development and homeostasis of multicellular organisms (H. Steller, Science 267, 1445-1449 (1995)). Derangements of apoptosis contribute to the pathogenesis of several human diseases including cancer, neurodegenerative disorders, and acquired immune deficiency syndrome (C.B. Thmpson, Science 267, 1456-1462 (1995)). Recently, much attention has focused on the signal transduction and biological function of two cell surface death receptors, Fas/APO-1 and TNFR-1 (J.L. Cleveland, et al., Cell 81, 479-482 (1995); A. Fraser, et al., Cell 85, 781-784 (1996); S. Nagata, et al., Science 267, 1449-56 (1995)). Both are members of the TNF receptor family which also include TNFR-2, low affinity NGFR, CD40, and CD30, among others (C.A. Smith, et al., Science 248, 1019-23 (1990); M. Tewari, et al., in Modular Texts in Molecular and Cell Biology M. Purton, Heldin, Carl, Ed. (Chapman and Hall, London, 1995). While family members are defined by the presence of cysteine-rich repeats in their extracellular domains, Fas/APO-1 and TNFR-1 also share an 80 amino acid region of intracellular homology, appropriately designated the "death domain", which is responsible for transducing signals for programmed cell death (Tartaglia et al., Cell 74:845 (1993); P. Golstein, et al., Cell 81, 185-6 (1995)). This shared death domain suggests that both receptors interact with a related set of signal transducing molecules that, until recently, remained unidentified. Using molecular interaction screens, several non-receptor cytoplasmic proteins which bind to the death domain of TNFR1 or Fas have been identified (reviewed in J.L. Cleveland and J.N. Ihle, Cell 81,479-482 (1995)). These proteins, FADD, TRADD, and RIP, also encode death domains, which mediate their interaction with the death domains of TNFR1 or Fas.

In addition to binding the death domain of the receptors, these cytoplasmic adaptor proteins also bind to proteins which link the receptors to pathways resulting in apoptosis or the production of inflammatory cytokines. For instance, upon activation of Fas, the cytoplasmic adaptor FADD binds to the death domain of Fas (Chinnaiyan et al., Cell 81, 505-512 (1995)). FADD recruits a cysteine protease-containing protein, FLICE, to the receptor complex via a homotypic interaction between death effector domains (DED) in FADD and FLICE (Muzio et al. Cell 85, 817-827 (1996)). This aggregation event leads to the proteolytic activation of FLICE, enabling it to cleave and activate other apoptotic cysteine proteases as well as poly ADP-ribose polymerase (PARP), an enzyme which exhibits a characteristic cleavage during apoptosis.

Similar to the association of FADD with Fas, TRADD associates with the death domain of TNFR1 upon receptor activation. TRADD then associates with FADD and FLICE to connect TNFR1 to the apoptotic pathway. However, overexpression of TRADD also activates the transcription factor NFkB, which in turn activates the transcription of inflammatory cytokines upon translocation to the nucleus (Hsu et al., Cell 81, 495-504 (1995)). TRADD appears to activate NFkB by binding to TRAF2, since dominant negative TRAF2 mutants block activation (Hsu et al., Cell 84, 299-308 (1996)).

RIP binds to the death domains of Fas and TRADD, thereby conferring signalling via both Fas and TNFR1 (Stanger et al, Cell 81, 513-523 (1995); Hsu et al, Immunity 4, 387-396 (1996)). RIP encodes a serine-threonine kinase and is capable of inducing both apoptosis and NFkB activation upon overexpression.

The effects of TNF family ligands, receptors, and their cytoplasmic adaptor molecules are varied and influence numerous functions, both normal and abnormal, in the biological processes of the mammalian system. There is a clear need, therefore, for identification and characterization of such ligands, receptors, and cytoplasmic adaptors that influence biological activity, both normally and in disease states.

This indicates that these death domain cytoplasmic adaptors have an established, proven history as therapeutic targets. Clearly, there is a need for identification and characterization of further members of death domain cytoplasmic adaptor family which can play a role in preventing, ameliorating or correcting dysfunctions or diseases, including, but not limited to, neurodegenerative disorders (such as Alzheimer's disease, Parkinson's disease, cerebellar degeneration, and amyotrophic lateral sclerosis), ischemic injury (such as stroke, myocardial infarction, and reperfusion injury), AIDS, aplastic anemia, polycistic kidney disease, osteoporosis, chronic degenerative liver disease, cancer, autoimmune disorders (such as systemic lupus erythematosus and immune-mediated glomerulonephritis), viral infections, and rheumatoid arthritis.

### SUMMARY OF THE INVENTION

In one aspect, the invention relates to DD-1 polypeptides and recombinant materials and methods for their production. Another aspect of the invention relates to methods for using such DD-1 polypeptides and polynucleotides. Such uses include the treatment of diseases associated with excess or inappropriate cell death, such as (but not limited to) neurodegenerative disorders (including Alzheimer's disease, Parkinson's disease, cerebellar degeneration, and amyotrophic lateral sclerosis), ischemic injury (including stroke, myocardial infarction, and reperfusion injury), AIDS, aplastic anemia, polycistic kidney disease, osteoporosis, and chronic degenerative liver disease. Additional uses include the treatment of diseases associated with an inhibition of apoptosis, such as (but not limited to) cancer, autoimmune disorders (including systemic lupus erythematosus and immune-mediated glomerulonephritis), and viral infections. Since other members of this family mediate the NFkB-dependent activation of inflammatory cytokines, another use may be the treatment of chronic inflammation associated with diseases such as rheumatoid arthritis, among others. In still another aspect, the invention relates to methods to identify agonists and antagonists using the materials provided by the invention, and treating conditions associated with DD-1 imbalance with the identified compounds. Yet another aspect of the invention relates to diagnostic assays for detecting diseases associated with inappropriate DD-1 activity or levels.

### BRIEF DESCRIPTION OF THE DRAWINGS

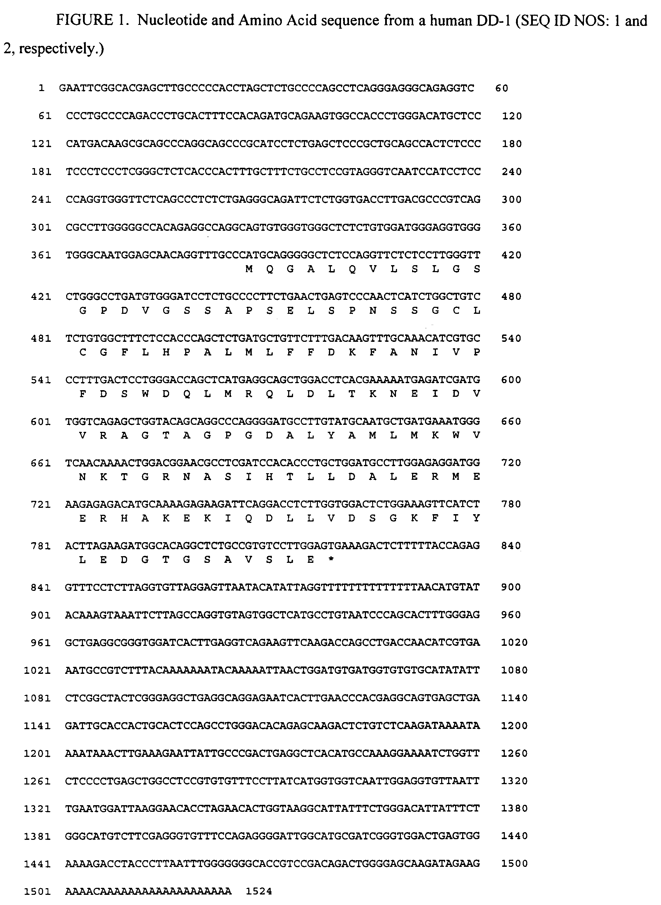

Figure 1 shows the nucleotide and deduced amino acid sequence from a human DD-1 (SEQ ID NOS: 1 and 2).

### DESCRIPTION OF THE INVENTION

### Definitions

The following definitions are provided to facilitate understanding of certain terms used frequently herein.

"DD-1 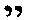 refers, among others, generally to a polypeptide having the amino acid sequence set forth in SEQ ID NO:2 or an allelic variant thereof.

"DD-1 activity or DD-1 polypeptide activity 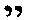 or 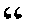 biological activity of the DD-1 or DD-1 polypeptide 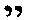 refers to the metabolic or physiologic function of said DD-1 including similar activities or improved activities or these activities with decreased undesirable side-effects. Also included are antigenic and immunogenic activities of said DD-1.

"DD-1 gene 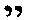 refers to a polynucleotide having the nucleotide sequence set forth in SEQ ID NO:1 or allelic variants thereof and/or their complements.

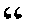 Antibodies 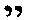 as used herein includes polyclonal and monoclonal antibodies, chimeric, single chain, and humanized antibodies, as well as Fab fragments, including the products of an Fab or other immunoglobulin expression library.

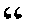 Isolated 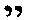 means altered 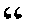 by the hand of man 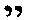 from the natural state. If an 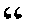 isolated 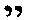 composition or substance occurs in nature, it has been changed or removed from its original environment, or both. For example, a polynucleotide or a polypeptide naturally present in a living animal is not 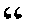 isolated, 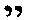 but the same polynucleotide or polypeptide separated from the coexisting materials of its natural state is 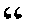 isolated 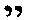 , as the term is employed herein.

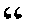 Polynucleotide 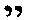 generally refers to any polyribonucleotide or polydeoxribonucleotide, which may be unmodified RNA or DNA or modified RNA or DNA. 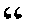 Polynucleotides 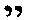 include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, 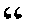 polynucleotide 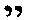 refers to triple-stranded regions comprising RNA or DNA or both RNA and DNA. The term polynucleotide also includes DNAs or RNAs containing one or more modified bases and DNAs or RNAs with backbones modified for stability or for other reasons. 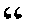 Modified 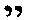 bases include, for example, tritylated bases and unusual bases such as inosine. A variety of modifications has been made to DNA and RNA; thus, 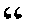 polynucleotide 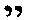 embraces chemically, enzymatically or metabolically modified forms of polynucleotides as typically found in nature, as well as the chemical forms of DNA and RNA characteristic of viruses and cells. 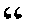 Polynucleotide 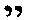 also embraces relatively short polynucleotides, often referred to as oligonucleotides.

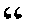 Polypeptide 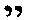 refers to any peptide or protein comprising two or more amino acids joined to each other by peptide bonds or modified peptide bonds, i.e., peptide isosteres. 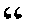 Polypeptide 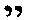 refers to both short chains, commonly referred to as peptides, oligopeptides or oligomers, and to longer chains, generally referred to as proteins. Polypeptides may contain amino acids other than the 20 gene-encoded amino acids. 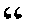 Polypeptides 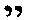 include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T.E. Creighton, W.H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., 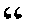 Analysis for protein modifications and nonprotein cofactors 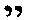 , *Meth Enzymol* (1990) 182:626-646 and Rattan *et al*., 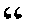 Protein Synthesis: Posttranslational Modifications and Aging 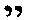 , *Ann NY Acad Sci* (1992) 663:48-62.

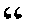 Variant 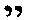 as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

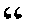 Identity 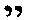 is a measure of the identity of nucleotide sequences or amino acid sequences. In general, the sequences are aligned so that the highest order match is obtained. 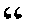 Identity 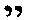 *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press; New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term 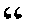 identity 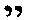 is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al*., *J Molec Biol* (1990) 215:403).

### Polypeptides of the Invention

In one aspect, the present invention relates to DD-1 polypeptides. The DD-1 polypeptides include the polypeptide of SEQ ID NO:2; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO:2. Also included within DD-1 polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Preferably DD-1 polypeptide exhibit at least one biological activity of DD-1.

The DD-1 polypeptides may be in the form of the 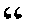 mature 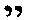 protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Biologically active fragments of the DD-1 polypeptides are also included in the invention. A fragment is a polypeptide having an amino acid sequence that entirely is the same as part, but not all, of the amino acid sequence of the aforementioned DD-1 polypeptides. As with DD-1 polypeptides, fragments may be 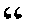 free-standing, 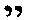 or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of DD-1 polypeptide. In this context 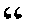 about 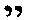 includes the particularly recited ranges larger or smaller by several, 5, 4, 3, 2 or 1 amino acid at either extreme or at both extremes.

Preferred fragments include, for example, truncation polypeptides having the amino acid sequence of DD-1 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Biologically active fragments are those that mediate DD-1 activity, including those with a similar activity or an improved activity, or with a decreased undesirable activity. Also included are those that are antigenic or immunogenic in an animal, especially in a human.

Preferably, all of these polypeptide fragments retain the biological activity of the DD-1, including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The DD-1 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to DD-1 polynucleotides. DD-1 polynucleotides include isolated polynucleotides which encode the DD-1 polypeptides and fragments, and polynucleotides closely related thereto. More specifically, DD-1 polynucleotide of the invention include a polynucleotide comprising the nucleotide sequence set forth in SEQ ID NO:1 encoding a DD-1 polypeptide of SEQ ID NO:2, and polynucleotide having the particular sequence of SEQ ID NO:1. DD-1 polynucleotides further include a polynucleotide comprising a nucleotide sequence that has at least 80% identity to a nucleotide sequence encoding the DD-1 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under DD-1 polynucleotides are a nucleotide sequence which has sufficient identity to a nucleotide sequence contained in SEQ ID NO:1 or contained in the cDNA insert in the plasmid deposited with the ATCC Deposit number ATCC98293 to hybridize under conditions useable for amplification or for use as a probe or marker. Moreover, DD-1 polynucleotide include a nucleotide sequence having at least 80% identity to a nucleotide sequence encoding the DD-1 polypeptide expressed by the cDNA insert deposited at the ATCC with Deposit Number ATCC98293, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above DD-1 polynucleotides.

A deposit containing a human DD-1 cDNA has been deposited with the American Type Culture Collection (ATCC), 12301 Park Lawn Drive, Rockville, Maryland 20852, USA, on 1/16/97, and assigned ATCC Deposit Number ATCC98293. The deposited material (clone) is E. coli XL-1 blue MRF containing pBluescriptSK(-) that further contains the full length DD-1 cDNA, referred to as 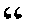 pBluescriptSK(-)-DD-1" upon deposit. The cDNA insert is within EcoRI-XhoI site(s) in the vector. The nucleotide sequence of the polynucleotides contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure. The strain will be irrevocably and without restriction or condition released to the public upon the issuance of a patent. The deposit is provided merely as convenience to those of skill in the art and is not an admission that a deposit is required for enablement, such as that required under 35 U.S.C. §112.

DD-1 of the invention is structurally related to other proteins of the death domain cytoplasmic adaptor, as shown by the results of sequencing the cDNA encoding human DD-1. The cDNA sequence contains an open reading frame encoding a polypeptide of 144 amino acids with a deduced molecular weight of **15.7** kDa. Amino acid of sequence of Figure 1 (SEQ ID NO:2) has about 18% identity (using Bestfit) in 77 amino acid residues with murine Fas antigen (R. Watanabe-Fukunaga et al., J. Immunol. 148:1274-1279, 1992).

One polynucleotide of the present invention encoding DD-1 may be obtained using standard cloning and screening, from a cDNA library derived from mRNA in cells of human bone marrow, tonsillar T-cells, and kidney using the expressed sequence tag (EST) analysis (Adams, M.D., *et al*. *Science* (1991) 252:1651-1656; Adams, M.D. *et al*., *Nature*, (1992) *355*:632-634; Adams, M.D., *et al*., *Nature* (1995) 377 Supp:3-174). Polynucleotides of the invention can also be obtained from natural sources such as genomic DNA libraries or can be synthesized using well known and commercially available techniques.

The nucleotide sequence encoding DD-1 polypeptide of SEQ ID NO:2 may be identical over its entire length to the coding sequence set forth in Figure 1 (SEQ ID NO:1), or may be a degenerate form of this nucleotide sequence encoding the polypeptide of SEQ ID NO:2, or may be highly identical to a nucleotide sequence that encodes the polypeptide of SEQ ID NO:2. Preferably, the polynucleotides of the invention comprise a nucleotide sequence that is highly identical, at least 80% identical, with a nucleotide sequence encoding a DD-1 polypeptide, or at least 80% identical with the sequence contained in Figure 1 (SEQ ID NO: 1) encoding DD-1 polypeptide, or at least 80% identical to a nucleotide sequence encoding the polypeptide of SEQ ID NO:2.

When the polynucleotides of the invention are used for the recombinant production of DD-1 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*., *Proc Natl Acad Sci USA* (1989) 86:821-824, or is an HA tag. The polynucleotide may also contain non-coding 5 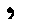 and 3 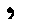 sequences, such as transcribed, non-translated sequences, splicing and polyadenylation signals, ribosome binding sites and sequences that stabilize mRNA.

Further preferred embodiments are polynucleotides encoding DD-1 variants comprise the amino acid sequence DD-1 polypeptide of Figure 1 (SEQ ID NO:2) in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term 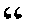 stringent conditions 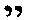 means hybridization will occur only if there is at least 95% and preferably at least 97% identity between the sequences.

Polynucleotides of the invention, which are identical or sufficiently identical to a nucleotide sequence contained in SEQ ID NO:1 or to the cDNA insert in the plasmid deposited at the ATCC with Deposit Number ATCC98293, may be used as hybridization probes for cDNA and genomic DNA, to isolate full-length cDNAs and genomic clones encoding DD-1 polypeptide and to isolate cDNA and genomic clones of other genes that have a high sequence similarity to the DD-1 gene. Such hybridization techniques are known to those of skill in the art. Typically these nucleotide sequences are 70% identical, preferably 80% identical, more preferably 90% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding DD-1 comprises the steps of screening an appropriate library under stingent hybridization conditions with a labeled probe having the SEQ ID NO: 1 or a fragment thereof, and isolating full-length cDNA and genomic clones containing said polynucleotide sequence. Such hybridization techniques are well known to those of skill in the art. Stringent hybridization conditions are as defined above or alternatively conditions under overnight incubation at 42^{o}C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65^{o}C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E*. *coli*, *Streptomyces and Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al*., *MOLECULAR CLONING*, *A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the DD-1 polypeptide is to be expressed for use in screening assays, generally, it is preferred that the polypeptide be produced at the surface of the cell. In this event, the cells may be harvested prior to use in the screening assay. If DD-1 polypeptide is secreted into the medium, the medium can be recovered in order to recover and purify the polypeptide; if produced intracellularly, the cells must first be lysed before the polypeptide is recovered. DD-1 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of DD-1 polynucleotides for use as diagnostic reagents. Detection of a mutated form of DD-1 gene associated with a dysfunction will provide a diagnostic tool that can add to or define a diagnosis of a disease or susceptibility to a disease which results from under-expression, over-expression or altered expression of DD-1. Individuals carrying mutations in the DD-1 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled DD-1 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. *See*, e.g., Myers *et al*., *Science* (1985) 230:1242. Sequence changes at specific locations may also be revealed by nuclease protection assays, such as RNase and S1 protection or the chemical cleavage method. See Cotton *et al*., *Proc Natl Acad Sci USA* (1985) 85: 4397-4401.

The diagnostic assays offer a process for diagnosing or determining a susceptibility to neurodegenerative disorders, aplastic anemia, polycistic kidney disease, osteoporosis, chronic degenerative liver disease, cancer, autoimmune disorders, and rheumatoid arthritis , among othersthrough detection of mutation in the **DD-1** gene by the methods described.

In addition, neurodegenerative disorders, aplastic anemia, polycistic kidney disease, osteoporosis, chronic degenerative liver disease, cancer, autoimmune disorders, and rheumatoid arthritis can be diagnosed by methods comprising determining from a sample derived from a subject an abnormally decreased or increased level of DD-1 polypeptide or DD-1 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an DD-1 polypeptide, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

### Antibodies

The polypeptides of the invention or their fragments or analogs thereof, or cells expressing them can also be used as immunogens to produce antibodies immunospecific for the DD-1 polypeptides. The term 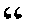 immunospecific 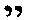 means that the antibodies have substantial greater affinity for the polypeptides of the invention than their affinity for other related polypeptides in the prior art.

Antibodies generated against the DD-1 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al*., *Immunology Today* (1983) 4:72) and the EBV-hybridoma technique (Cole *et al*., MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against **DD-1** polypeptides may also be employed to treat neurodegenerative disorders, aplastic anemia, polycistic kidney disease, osteoporosis, chronic degenerative liver disease, cancer, autoimmune disorders and rheumatoid arthritis, among other diseases and disorders.

### Vaccines

Another aspect of the invention relates to a method for inducing an immunological response in a mammal which comprises inoculating the mammal with DD-1 polypeptide, or a fragment thereof, adequate to produce antibody and/or T cell immune response to protect said animal from neurodegenerative disorders, aplastic anemia, polycistic kidney disease, osteoporosis, chronic degenerative liver disease, cancer, autoimmune disorders, and rheumatoid arthritis, among other diseases and disorders. Yet another aspect of the invention relates to a method of inducing immunological response in a mammal which comprises, delivering DD-1 polypeptide via a vector directing expression of DD-1 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a DD-1 polypeptide wherein the composition comprises a DD-1 polypeptide or DD-1 gene. The vaccine formulation may further comprise a suitable carrier. Since DD-1 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The DD-1 polypeptide of the present invention may be employed in a screening process for compounds which activate (agonists) or inhibit activation of (antagonists, or otherwise called inhibitors) the DD-1 polypeptide of the present invention. Thus, polypeptides of the invention may also be used to assess identify agonist or antagonists from, for example, cells, cell-free preparations, chemical libraries, and natural product mixtures. These agonists or antagonists may be natural substrates, ligands, receptors, etc., as the case may be, of the polypeptide of the present invention; or may be structural or functional mimetics of the polypeptide of the present invention. *See* Coligan *et al*., *Current Protocols in Immunology* 1(2):Chapter 5 (1991).

DD-1 polypeptides are ubiquitous in the mammalian host and are responsible for many biological functions, including many pathologies. Accordingly, it is desirous to find compounds and drugs which stimulate DD-1 polypeptide on the one hand and which can inhibit the function of DD-1 polypeptide on the other hand. In general, agonists are employed for therapeutic and prophylactic purposes for such conditions as neurodegenerative disorders, aplastic anemia, polycistic kidney disease, osteoporosis, chronic degenerative liver disease, cancer, autoimmune disorders, or rheumatoid arthritis. Antagonists may be employed for a variety of therapeutic and prophylactic purposes for conditions associated with perturbations in apoptosis or inflammation, such as neurodegenerative disorders (including Alzheimer's disease, Parkinson's disease, cerebellar degeneration, and amyotrophic lateral sclerosis), ischemic injury (including stroke, myocardial infarction, and reperfusion injury), AIDS, aplastic anemia, polycistic kidney disease, osteoporosis, chronic degenerative liver disease, cancer, autoimmune disorders (including systemic lupus erythematosus and immune-mediated glomerulonephritis), viral infections, and rheumatoid arthritis.

In general, such screening procedures may involve producing appropriate cells which express the DD-1 polypeptide of the present invention on the surface thereof. Such cells include cells from mammals, yeast, *Drosophila* or *E*. *coli*. Cells expressing the DD-1 polypeptide (or cell membrane containing the expressed polypeptide) are then contacted with a test compound to observe binding, or stimulation or inhibition of a functional response. The ability of the cells which were contacted with the candidate compound is compared with the same cells which were not contacted for DD-1 activity.

One example of such a screening strategy would be a yeast two-hybrid screen (C. Bai and S.J. Elledge, Meth. Enz. 273:331-347, 1996) in which the interaction of DD-1 with its ligand results in the activation of a reporter gene, typically β-galactosidase. This system could be screened for compounds which inhibit the interaction of DD-1 and its ligand, indicated by the absence of reporter gene expression.

The assays may simply test binding of a candidate compound wherein adherence to the cells bearing the DD-1 polypeptide is detected by means of a label directly or indirectly associated with the candidate compound or in an assay involving competition with a labeled competitor. Further, these assays may test whether the candidate compound results in a signal generated by activation of the DD-1 polypeptide, using detection systems appropriate to the cells bearing the DD-1 polypeptide at their surfaces. Inhibitors of activation are generally assayed in the presence of a known agonist and the effect on activation by the agonist by the presence of the candidate compound is observed. Standard methods for conducting such screening assays are well understood in the art.

Examples of potential DD-1 polypeptide antagonists include antibodies or, in some cases, oligonucleotides or proteins which are closely related to the ligands, substrates, receptors, etc., as the case may be, of the DD-1 polypeptide, e.g., a fragment of the ligands, substrates, receptors, or small molecules which bind to the polypetide of the present invention but do not elicit a response, so that the activity of the polypeptide is prevented.

### Prophylactic and Therapeutic Methods

This invention provides methods of treating an abnormal conditions related to both an excess of and insufficient amounts of DD-1 polypeptide activity.

If the activity of DD-1 polypeptide is in excess, several approaches are available. One approach comprises administering to a subject an inhibitor compound (antagonist) as hereinabove described along with a pharmaceutically acceptable carrier in an amount effective to inhibit activation by blocking binding of ligands to the DD-1 polypeptide, or by inhibiting a second signal, and thereby alleviating the abnormal condition.

In another approach, soluble forms of DD-1 polypeptides still capable of binding the ligand in competition with endogenous DD-1 polypeptide may be administered. Typical embodiments of such competitors comprise fragments of the DD-1 polypeptide.

In still another approach, expression of the gene encoding endogenous DD-1 polypeptide can be inhibited using expression blocking techniques. Known such techniques involve the use of antisense sequences, either internally generated or separately administered. See, for example, O 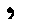 Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al*., *Nucleic Acids Res* (1979) 6:3073; Cooney *et* *al*., *Science* (1988) 241:456; Dervan *et al*., *Science* (1991) 251:1360. These oligomers can be administered *per se* or the relevant oligomers can be expressed *in vivo*.

For treating abnormal conditions related to an under-expression of DD-1 and its activity, several approaches are also available. One approach comprises administering to a subject a therapeutically effective amount of a compound which activates DD-1 polypeptide, i.e., an agonist as described above, in combination with a pharmaceutically acceptable carrier, to thereby alleviate the abnormal condition. Alternatively, gene therapy may be employed to effect the endogenous production of DD-1 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo*. For overview of gene therapy, see Chapter 20, *Gene Therapy and other Molecular Genetic*-*based Therapeutic Approaches*, (and references cited therein) in Human Molecular Genetics, T Strachan and A P Read, BIOS Scientific Publishers Ltd (1996).

### Formulation and Administration

Peptides, such as the soluble form of DD-1 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject 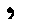 s condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as 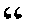 gene therapy 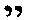 as described above. Thus, for example, cells from a subject may be engineered with a polynucleotide, such as a DNA or RNA, to encode a polypeptide *ex vivo*, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1: Isolation and identification of DD-1

A partial clone (HGS EST # 1324054) was initially identified through a search a database comprising a collection of sequenced human ESTs (Adams, M.D., *supra*) for proteins with homology to the death domain. This partial clone (512 bp) showed homology (18% over 77 aa) to murine Fas (Apo-1/CD-95). A PCR product (corresponding to nucleotides 204-487 of SEQ ID NO: 1) was generated from genomic DNA and used to screen 1 M. plaques from a human bone marrow cDNA library. The cDNA library screening procedure is described (J. Sambrook, E.F. Fritch and T. Maniatis (1989) A Laboratory Manaul Second. Ed. Vol. 1 pp. 2.108-2.117, Cold Spring Harbor Laboratory Press, Cold Spring Harbor, New York). The probes were α-32P labeled, using a Prime-It II Kit (Stratagene, La Jolla, CA) and purified by running over BioSpin 30 columns (BioRad Laboratories, Melville, NY). The hybridization and washing conditions were according to (J. Sambrook, E.F. Fritch and T. Maniatis (1989) A Laboratory Manaul Second. Ed. Vol. 1 pp. 2.69-2.81, Cold Spring Harbor Laboratory Press, Cold Spring Harbor, New York). Five independent positive phage clones were purified , and the insert-containing pBluescriptSK(-) plasmid was rescued from each using a Rapid Excision Kit (Stratgene, La Jolla, CA). Upon digestion with EcoRI and XhoI, each of the 5 independent cDNA clones released the same 1.5 kb insert, suggesting that they are identical. Two of these cDNA inserts were sequenced completely on both strands using an automated sequencer. A total of 1524 bp were sequenced, and this includes an open reading frame encoding a peptide of 144 aa. The cDNA and protein sequences are SEQ ID NOS: 1 and 2, respectively, and are named Death Domain-1 (DD-1).

## Claims

1. An isolated polynucleotide comprising a nucleotide sequence that has at least 80% identity to a nucleotide sequence encoding the DD-1 polypeptide of SEQ ID NO:2 over its entire length; or a nucleotide sequence complementary to said nucleotide sequence.

2. The polynucleotide of claim 1 which is DNA or RNA.

3. The polynucleotide of claim 1 wherein said nucleotide sequence is at least 80% identical to that contained in SEQ ID NO:1.

4. The polynucleotide of claim 3 wherein said nucleotide sequence comprises the DD-1 polypeptide encoding sequence contained in SEQ ID NO:1.

5. The polynucleotide of SEQ ID NO: 1.

6. A polynucleotide probe or primer comprising at least 15 contiguous nucleotides of the polynucleotide of claim 3.

7. A DNA or RNA molecule comprising an expression system, wherein said expression system is capable of producing a DD-1 polypeptide comprising an amino acid sequence which has at least 80% identity with the polypeptide of SEQ ID NO:2 when said expression system is present in a compatible host cell.

8. A host cell comprising the expression system of claim 7.

9. A process for producing a DD-1 polypeptide comprising culturing a host of claim 8 and under conditions sufficient for the production of said polypeptide.

10. The process of claim 9 wherein said polypeptide is expressed at the surface of said cell.

11. The process of claim 9 which further includes recovering the polypeptide from the culture.

12. A process for producing a cell which produces a DD-1 polypeptide thereof comprising transforming or transfecting a host cell with the expression system of claim 7 such that the host cell, under appropriate culture conditions, produces a DD-1 polypeptide.

13. Cells produced by the process of claim 12.

14. A DD-1 polypeptide comprising an amino acid sequence which is at least 80% identical to the amino acid sequence of SEQ ID NO:2 over its entire length.

15. The polypeptide of claim 14 which comprises the amino acid sequence of SEQ ID NO:2.

16. The polypeptide of SEQ ID NO: 2.

17. A DD-1 polypeptide prepared by the method of claim 11.

18. An antibody immunospecific for the DD-1 polypeptide of claim 14.

19. A method for the treatment of a subject in need of enhanced DD-1 activity comprising:
(a) administering to the subject a therapeutically effective amount of an agonist to said receptor; and/or
(b) providing to the subject DD-1 polynucleotide in a form so as to effect production of said receptor activity *in vivo*.

20. A method for the treatment of a subject having need to inhibit DD-1 activity comprising:
(a) administering to the subject a therapeutically effective amount of an antagonist to said receptor; and/or
(b) administering to the subject a nucleic acid molecule that inhibits the expression of the nucleotide sequence encoding said receptor; and/or
(c) administering to the subject a therapeutically effective amount of a polypeptide that competes with said receptor for its ligand.

21. A process for diagnosing a disease or a susceptibility to a disease in a subject related to expression or activity of DD-1 in a subject comprising:
(a) determining the presence or absence of a mutation in the nucleotide sequence encoding said DD-1 in the genome of said subject; and/or
(b) analyzing for the presence or amount of the DD-1 expression in a sample derived from said subject.

22. A method for identifying compounds which bind to DD-1 polypeptide comprising:
(a) contacting cells of claim 13 with a candidate compound; and
(b) assessing the ability of said candidate compound to bind to said cells.

23. The method of claim 22 which further includes determining whether the candidate compound effects a signal generated by activation of the DD-1 polypeptide at the surface of the cell, wherein a candidate compound which effects production of said signal is identified as an agonist.

24. An agonist identified by the method of claim 23.

25. The method of claim 22 which further includes contacting said cell with a known agonist for said DD-1 polypeptide; and
determining whether the signal generated by said agonist is diminished in the presence of said candidate compound, wherein a candidate compound which effects a diminution in said signal is identified as an antagonist for said DD-1 polypeptide.

26. An antagonist identified by the method of claim 25.

27. A polynucleotide consisting essentially of a DNA sequence obtainable by screening an appropriate library containing the DD-1 gene under stringent hybridization conditions with a probe having the sequence of SEQ ID NO: 1 or a fragment thereof; and isolating said DNA sequence.

28. A polypetide obtainable by expressing a nucleotide sequence comprising that of SEQ ID NO:1.

29. An isolated DD-1 polynucleotide comprising a nucleotide sequence selected from the group consisting of:
(a) a nucleotide sequence having at least 80% identity to a nucleotide sequence encoding the DD-1 polypeptide expressed by the cDNA insert deposited at the ATCC with Deposit Number ATCC98293;
(b) a nucleotide sequence complementary to the nucleotide sequence of (a); and
(c) a nucleotide sequence comprising at least 15 contiguous nucleotides of the nucleotide sequence of (a) or (b).


SEQUENCE LISTING

(1) GENERAL INFORMATION

(i) APPLICANT: SmithKline Beecham Corporation

(ii) TITLE OF THE INVENTION: Novel Mammalian DD-1 Gene

(iii) NUMBER OF SEQUENCES: 2

(iv) CORRESPONDENCE ADDRESS:
(A) ADDRESSEE: F J Cleveland & Company
(B) STREET: 40/43 Chancery Land
(C) CITY: London
(D) STATE:
(E) COUNTRY: United Kingdom
(F) POST CODE: WC2A 1JQ

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER:
(B) FILING DATE: 27-JAN-1997
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: CRUMP, Julian Richard John
(B) REGISTRATION NUMBER: 37127
(C) REFERENCE/DOCKET NUMBER: ATG50049

(ix) TELECOMMUNICATION INFORMATION:
(A) TELEPHONE: +44 171 405 5875
(B) TELEFAX: +44 171 831 0749
(C) TELEX:

(2) INFORMATION FOR SEQ ID NO: 1:

(i) SEQUENCE CHARACTERISTICS:
(A) LENGTH: 1524 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 1:

GAATTCGGCA CGAGCTTGCC CCCACCTAGC TCTGCCCCAG CCTCAGGGAG GGCAGAGGTC 60
CCCTGCCCCA GACCCTGCAC TTTCCACAGA TGCAGAAGTG GCCACCCTGG GACATGCTCC 120
CATGACAAGC GCAGCCCAGG CAGCCCGCAT CCTCTGAGCT CCCGCTGCAG CCACTCTCCC 180
TCCCTCCCTC GGGCTCTCAC CCACTTTGCT TTCTGCCTCC GTAGGGTCAA TCCATCCTCC 240
CCAGGTGGGT TCTCAGCCCT CTCTGAGGGC AGATTCTCTG GTGACCTTGA CGCCCGTCAG 300
CGCCTTGGGG GCCACAGAGG CCAGGCAGTG TGGGTGGGCT CTCTGTGGAT GGGAGGTGGG 360
TGGGCAATGG AGCAACAGGT TTGCCCATGC AGGGGGCTCT CCAGGTTCTC TCCTTGGGTT 420
CTGGGCCTGA TGTGGGATCC TCTGCCCCTT CTGAACTGAG TCCCAACTCA TCTGGCTGTC 480
TCTGTGGCTT TCTCCACCCA GCTCTGATGC TGTTCTTTGA CAAGTTTGCA AACATCGTGC 540
CCTTTGACTC CTGGGACCAG CTCATGAGGC AGCTGGACCT CACGAAAAAT GAGATCGATG 600
TGGTCAGAGC TGGTACAGCA GGCCCAGGGG ATGCCTTGTA TGCAATGCTG ATGAAATGGG 660
TCAACAAAAC TGGACGGAAC GCCTCGATCC ACACCCTGCT GGATGCCTTG GAGAGGATGG 720
AAGAGAGACA TGCAAAAGAG AAGATTCAGG ACCTCTTGGT GGACTCTGGA AAGTTCATCT 780
ACTTAGAAGA TGGCACAGGC TCTGCCGTGT CCTTGGAGTG AAAGACTCTT TTTACCAGAG 840
GTTTCCTCTT AGGTGTTAGG AGTTAATACA TATTAGGTTT TTTTTTTTTT TAACATGTAT 900
ACAAAGTAAA TTCTTAGCCA GGTGTAGTGG CTCATGCCTG TAATCCCAGC ACTTTGGGAG 960
GCTGAGGCGG GTGGATCACT TGAGGTCAGA AGTTCAAGAC CAGCCTGACC AACATCGTGA 1020
AATGCCGTCT TTACAAAAAA ATACAAAAAT TAACTGGATG TGATGGTGTG TGCATATATT 1080
CTCGGCTACT CGGGAGGCTG AGGCAGGAGA ATCACTTGAA CCCACGAGGC AGTGAGCTGA 1140
GATTGCACCA CTGCACTCCA GCCTGGGACA CAGAGCAAGA CTCTGTCTCA AGATAAAATA 1200
AAATAAACTT GAAAGAATTA TTGCCCGACT GAGGCTCACA TGCCAAAGGA AAATCTGGTT 1260
CTCCCCTGAG CTGGCCTCCG TGTGTTTCCT TATCATGGTG GTCAATTGGA GGTGTTAATT 1320
TGAATGGATT AAGGAACACC TAGAACACTG GTAAGGCATT ATTTCTGGGA CATTATTTCT 1380
GGGCATGTCT TCGAGGGTGT TTCCAGAGGG GATTGGCATG CGATCGGGTG GACTGAGTGG 1440
AAAAGACCTA CCCTTAATTT GGGGGGGCAC CGTCCGACAG ACTGGGGAGC AAGATAGAAG 1500
AAAACAAAAA AAAAAAAAAA AAAA 1524

(2) INFORMATION FOR SEQ ID NO: 2:

(i) SEQUENCE CHARACTERISTICS:

(A) LENGTH: 144 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 2: